# EUROPEAN PATENT SPECIFICATION

(11) **EP 0 344 320 B1**
(45) Date of publication and mention of the grant of the patent: **29.11.1995**
(21) Application number: 88908988.4
(22) Date of filing: 13.10.1988
(51) Int. Cl.: C07K 16/00, C12N 5/00, C12N 15/00, C12P 21/00, A61K 39/395, G01N 33/577

(54) **HUMAN MONOCLONAL ANTIBODY AGAINST CANDIDA**
MENSCHLICHER MONOKLONALER ANTIKÖRPER GEGEN CANDIDA
ANTICORPS MONOCLONAL HUMAIN CONTRE LE CANDIDA

(30) Priority: 14.10.1987 JP 257431/87
(43) Date of publication of application: 06.12.1989
(73) Proprietor: TEIJIN LIMITED, Osaka-shi Osaka 541 (JP)
(72) Inventor: KAWAMURA, Takashi, Tucson, AZ 85718 (US); MASUHO, Yasuhiko Teijin Shataku C, Tokyo 191 (JP); SAWADA, Shuzo, Tokyo 191 (JP); SASAKI, Satoshi, Tokyo 191 (JP)
(74) Representative: Cookson, Barbara Elizabeth
(86) International application number: JP8801042
(87) International publication number: WO8903398

(56) References cited:
- EP-A- 0 233 289
- WO-A-86/02365
- JP-A- 6 262 000
- BIOTECHNOLOGY, vol. 4, no. 3, March 1986, pages 210-218, New York, US; N. CHIORAZZI: "Human monoclonal antibodies as probes to study autoimmune and allergic disorders"
- INFECTION AND IMMUNITY, vol. 43, no. 3, March 1984, pages 966-972, American
- Society for Microbiology; D.L. BRAWNER et al.: "Variability in expression of a cell surface determinant on Candida albicans as evidenced by an agglutinating monoclonal antibody"
- Sawada, S. et al (1985) J.Infect. Dis. 152:965-970
- Miyakawa, Y. et al (1986) J. Clin. Microbiol. 23:881-886

## Description

### TECHNICAL FIELD

The present invention relates to human monoclonal antibodies to Candida and hybridoma producing same, and a process for production of said monoclonal antibodies. These monoclonal antibodies are useful for the diagnosis and treatment of Candida infection diseases.

### BACKGROUND ART

Candida is a genus belonging to fungi, including the species Candida albicans A, C. albicans B, C. tropicalis, C. stellatoidea, C. guilliermondii, C. Krusei, C. parapsilosis, C. kefyr, and C. glabrate. Among the above, C. albicans is important as an opportunistic pathogen which causes severe infection in a patient suffering from immunodeficiency. Although these fungi are common microorganisms found in the digestive tract and cavities of 10 to 50% of a normal human, upon a decreased resistance to infection in the host, an administration of antibiotics for a long term or upon a surgical invasion, these microorganisms abnormally proliferate, damage tissues, and can enter the blood. For such deep seated fungal infection, the diagnosis and treatment of Candida is therapeutically important.

For example, in the case of a patient suffering from an immunodeficiency and having a persistent fever which resists usual therapy, deep seated candidiasis must be suspected. To diagnose the candidiasis, it is important to determine the presence of lesions in the retina by a fundoscopy. Alternatively, a blood, abscess, urine, bioptic sample or the like is cultured in various media, and the morphology of the cultured microorganisms are observed, or a sugar-fermentation test or serological identification is carried out.

For a rapid and accurate serological diagnosis of Candida, preferably a monoclonal antibody having an antibody titer and a specificity higher than a serum antibody is used. To date, anti-Candida monoclonal antibodies of mouse origin have been described. D.L. Brawner and J.E. Cutler (Infect. Immun. 43, 966, 1984) prepared a monoclonal antibody which agglutinates C. albicans, C. tropicalis and C. glabrata among Candida; N.A. Strockbine et al (Infect. Immun. 43, 1012, 1984) prepared a monoclonal antibody to protein of C. albicans; and Y. Miyakawa et al (J. Clin. Microbiol. 23, 881, 1986) prepared a monoclonal antibody to mannan antigen of Candida. All of these reported monoclonal antibodies are of mouse origin, not of human origin. Human monoclonal antibodies are advantageous in that the F(ab')₂ fragment thereof is more easily obtained than that of a mouse monoclonal antibody, leading to an easy production of a diagnostic agent, wide availability of cysteine residue, and a decreased non-specific adsorption by Fc moiety.

Where systemic candidiasis is complicated in an immunodeficiency patient, an antifungal agent is systemically administered. As antifungal agents, Amphotericin B and Flucytosine are currently used, and Ketoconazole, Miconazole and the like are now being developed. Although Amphotericin is a pharmaceutical primarily selected against fatal candidiasis, it causes side effects such as fever, nephropathy and marrow disorder. Although Flucytosine has a lower toxicity than Amphotericin, it is less effective against candidiasis. Ketoconazole and Miconazole cause severe side effects. Considering the above situation in the art, pharmaceuticals having a greater effect against candidiasis but lower side effects, is desired.

As is obvious from the fact that Candida is common microorganism and its infection is caused by an immunodeficiency, the immunological competence of a human functions as an effective defence mechanism. The immune system is roughly classified into cell-mediated immunity and humoral immunity. N.N. Pearsall et al. immunized an animal with Candida, removed immunocytes and immune serum from the animal, and compared the immunocytes and immune serum in their defensive effect against an animal having candidiasis, and reported that the immune serum was more effective (J. Immunol. 120, 1176, 1978). An essential entity as a defensive factor in an immune serum is an immunoglobulin (abbreviated Ig , also called antibody). Accordingly, a prophylaxis and treatment of candidiasis should be possible by adminiserting an antibody to Candida to a patient suffering from immunodeficiency. Although commercial normal human serum globulin preparations which are currently available contain antibodies to Candida, the content thereof is very low, and due to this low content, the defensive effect against Candida is naturally very low. Considering the aspect of an antibody titer, a monoclonal antibody is an antibody consisting of a single molecular species, and its titer is higher than that of antibodies present in an immune serum. Moreover, a large amount of monoclonal antibody can be produced by a proliferation of monoclonal antibody-producing cells. Although monoclonal antibodies to Candida have been described, since these monoclonal antibodies are mouse protein derived from mouse lymphocytes, when administered to a human it is recognized as a foreigner and an immune reaction to mouse protein occurs, resulting in an inhibition of an immune activity of the mouse monoclonal antibody, and further, the mouse antibody provides unfavorable side effects due to an antigen-antibody complex. Accordingly there is a need to develop anti-Candida monoclonal antibodies of human origin useful for a prophylaxis and treatment of Candida infection.

Generally, a monoclonal antibody of human origin is produced by hybridoma obtained by cell fusion between mouse myeloma cell, human myeloma cell or other established lymphocyte and human lymphocyte. Alternatively, it is produced by lymphoblastoid cells transformed with EB virus. Although from 1980 onward the production of many human monoclonal antibodies has been attempted, each process has characteristic problems. The production of a monoclonal antibody by hybridoma obtained from cell fusion between mouse myeloma cell and human lymphocyte is unstable, and the efficiency of a cell fusion between human myeloma cell and human lymphocyte is very low.

Moreover, the production of a monoclonal antibody by lymphoblastoid obtained using an EB virus has a low yield and is unstable. In addition, since the EB virus can generate tumors, problems arise from the point of view of safety.

As described above, there are large obstacles to the establishment of a human monoclonal antibody-producing cell. Moreover, there is a problem of how to obtain a human lymphocyte immunized against Candida. Human lymphocytes can be obtained from peripheral blood; a surgically removed spleen, lymph nodes or tonsils; or from marrow. Generally, although peripheral blood is readily available, the availability of other tissues is very limited. Moreover, in general, peripheral blood contains a small amount of B lymphocytes, and therefore, a very small amount of B lymphocytes activated by an antigen, and a large portion of such cells gather in the lymphatic tissues. Accordingly, it is difficult to obtain Candida-specific B cells from peripheral blood. Even where the solid lymphatic tissues are used, the availability of a lymphocyte-donor sufficiently immunized against Candida is not high. Accordingly, due to the above-mentioned obstacles, human monoclonal antibodies to Candida have never been produced.

### DISCLOSURE OF THE INVENTION

Accordingly, the present invention provides a hybridoma capable of producing a human monoclonal antibody against Candida characterised in that the hybridoma is obtainable by the process comprising the steps of obtaining human B-lymphocytes, screening the lymphocytes for anti-Candida activity, selecting the lymphocytes with high anti-Candida activity, fusing said lymphocytes with myeloma cells to form hybridomas, screening the hybridomas for high antibody production and cloning said hybridomas to obtain hybridomas capable of producing anti-Candida monoclonal antibody, and in that the human monoclonal antibody produced therefrom has an ability to agglutinate Candida cells and is reactive with the Candida cell wall polysaccharide, mannan.

The present invention also provides a human monoclonal antibody to Candida produced by such a hybridoma, in which the human-B-lymphocytes are selected from human tonsils, spleens, adenoids or lymph nodes.

### Brief Explanation of the Drawings

Figure 1 is a graph showing how an antigen-antibody reaction between various monoclonal antibodies of the present invention and C. albicans A is inhibited by mannan; and
Fig. 2 is a graph showing how an antigen-antibody reaction between various monoclonal antibodies of the present invention and C. albicans B is inhibited by mannan.

### Best Mode of Carrying Out the Invention

According to the present invention, first a human B-lymphocyte capable of producing an antibody to Candida is obtained, and a cell line producing a human anti-Candida antibody is established. According to the procedure, a human B-lymphocyte capable of producing a monoclonal antibody to Candida is, optionally after stimulation with a B-lymphocyte activating factor, fused with a myeloma cell (cell fusion partner). After about 3 to 5 weeks from the fusion, supernatants of developed hybridomas are sampled and tested for the presence of an antibody to Candida. Hybridomas producing a specific antibody are then cloned, and clones exhibiting an excellent antibody producing ability are selected. Where a tonsil cell having a high ability to produce an antibody to Candida is used, although clones producing a Candida-specific antibody are efficiently developed, since a major portion thereof is unstable in antibody production, they must be cloned at a precise timing with the addition of feeder cells. The cells thus obtained, producing a human anti-Candida monoclonal antibody, are cultured, and a supernatant thereof is collected which is then subjected to column chromatography or the like to purify the human monoclonal antibody.

Lymphocytes used in the present invention are contained in the human spleen, lymph node, tonsils, adenoids, marrow, peripheral blood, and the like. Although lymphocytes from any source are used for the present invention, preferably, these lymphocytes are derived from a solid lymphoid tissue such as the tonsil, adenoid, spleen, or lymph node. Most preferably, a source is used which provides a lymphocyte having an ability to produce a large amount of antibodies to Candida, when cultured in vitro with a lymphocyte activating factor and/or a Candida antigen.

For example, first, many human tonsils removed from patients suffering from tonsilitis are collected, and lymphocytes are separated from the collected tonsils. The immune response to Candida varies remarkably, depending on the tonsil donors, and accordingly, lymphocyte sources are screened for a high ability to produce in vitro an antibody to Candida. In this manner, a lymphocyte source having the highest ability to produce an antibody to Candida is obtained.

Where lymphocytes obtained from the thus selected lymphocyte source are used to construct hybridoma, preferably prior to the cell fusion, the lymphocytes are stimulated with a B-lymphocyte activating factor and/or a Candida antigen. The B-lymphocyte activating factor may be any substance which promotes the growth of B-lymphocyte, and includes, for example, pokeweed mitogen (PWM), B-cell growth factors (BCGF), protein A, phytohaemaglutinin (PHA) and concanavalin A. Preferably, 2 to 200 »g/ml PWM or one hundredth to one third volume of BCGF is used. As the Candida antigen, Candida cells per se or an antigen extracted from Candida cells, such as mannan, is used. The cells are preferably used in an amount of 1 x 10⁵ to 1 x 10⁸ cells/ml, and mannan is preferably added in the form of a complex with methylated bovine serum albumin to a concentration of 10 ng/ml to 1 ng/ml. Although the method and condition of stimulation are not critical, for example, one hundredth to one third volume of BCGF and 1 x 10⁵ to 1 x 10⁷ lymphocytes are mixed, and cultured. The culturing temperature is 35°C to 40°C, and the culturing time is 3 to 10 days, preferably 4 to 6 days. Although any culture medium containing serum of human, bovine or horse origin may be used, media, such as RPMI 1640, containing fetal calf serum (FCS) are particularly preferable.

Myeloma cells used in the present invention are human myeloma cells, human B-lymphoblasts, human B-lymphoma cells, mouse myeloma cells, hetero-myeloma cells derived from hybridoma of human lymphocyte and mouse myeloma cell, or the like. As mouse myeloma cell lines exhibiting an especially high fusion efficiency, P3 x 63Ag 8; P3-NS1/1-Ag4-1; P3 x 63AgUl; SP2/OAg 14; P3 X 62Ag 8, 6, 5 and 3; MPC11-45, 6, TG1.7; SP-1, and the like are preferable, although fundamentally any myeloma cell line can be used in the present invention.

The fusion between human lymphocyte and myeloma cell is carried out as follows. For example, antibody-producing cells and myeloma cells are mixed at a ratio of 10:1 to 1:10, preferably 1:1 to 1:3, to the mixture is added an appropriate cell fusion medium such as RPMI 1640 containing about 35% polyethylene glycol (approximate molecular weight 1,000 to 6,000) and about 7.5% dimethylsulfoxide, the mixture is stirred at a temperature between room temperature and 37°C for 1 to several minutes, gradually diluted with RPMI 1640 supplemented with 10% FCS, and the cell concentration is adjusted to 1 to 5 x 10⁵ cells/ml by a HAT (hypoxanthine-aminopterin-thymidine) selection medium. Then 0.2 ml of the mixture is distributed, for example, to each well of a 96-well plate, and cultured in air containing 5% CO₂ , at 35°C to 38°C for 2 to 3 weeks. In the HAT medium, only hybridoma survives, and myeloma cells which are resistant to 8-azaguanine and myeloma X myeloma fused cells cannot survive (non-fused antibody-producing cells die in a few days).

After culturing, the antibody titer of the culture broths is checked, to select and isolate hybridomas producing a desired antibody (cloning). This check of the antibody titer in a culture broth can be carried out by a radioimmunoassay (RIA), Enzyme-linked immunosorbent assay (ELISA), cell agglutination technique. The hybridoma producing the human anti-Candida monoclonal antibody of the present invention, selected by the cloning, can be frozen and stored. Such a hybridoma cell line and/or cell line derived therefrom is cultured on a large scale to obtain a desired human monoclonal antibody from the culture supernatant. Alternatively, hybridoma can be transplanted to an animal to form a tumor, and a monoclonal antibody can be obtained from ascites or serum of the animal.

Human anti-Candida monoclonal antibodies obtained as described above have the following properties. The human anti-Candida monoclonal antibodies of the present invention are of the class IgG, IgM, or IgA.

The human monoclonal antibodies obtained according to the present invention commonly react with many experimental Candida strains and clinical isolants. As the Candida strains, there can be mentioned C. albicans A, C. albicans B, C. tropicalis, C. stellatoidea, C. gulliermondii, C. Krusei, C. parapsilosis, C. kefyr, and C. glabrate. Human monoclonal antibodies obtained according to the present invention have different reactivities, and thus some react with a wide spectrum of the above-mentioned strains, and others react with only a few of the strains.

Human monoclonal antibodies obtained according to the present invention have an activity to agglutinate Candida cells.
Generally, a monoclonal antibody having the agglutinating activity is advantageous for both diagnostic and therapeutic purposes, because a diagnosis can be easily carried out by cell agglutination, and generally, a monoclonal antibody having an agglutination ability will strongly bind to cells, resulting in a strong defensive ability.

A Candida cell comprises many antigenic substances; specifically, mannan, proteins, and glucans are present in the cell surface. Especially, mannan present in the cell wall is important for diagnostic and therapeutic proposes. The human monoclonal antibodies obtained according to the present invention react with mannan.

For diagnostic and therapeutic purposes, the human monoclonal antibodies of the present invention have an activity to agglutinate Candida cells, react with mannan present in the cell wall and are preferably IgG.

The present human monoclonal antibodies are used for the diagnosis, prophylaxis, and/or treatment, of candidiasis.

Next, the present invention is explained in detail by Examples

### Example 1

### (1) Culturing of Candida

A piece of cells of C. albicans A (J 1012) or C. albicans B (J 1445) was taken from a slant culture and inoculated to 100 ml of a medium (0.5% yeast extract, 1% Polypepton and 2.0% glucose), and aerobically cultured at 30°C overnight with shaking. To the culture broth was added 35% formalin to make a final formaldehyde concentration of 1%, and a reaction was carried out at a room temperature for one hour. The cell suspension was then centrifuged at 2000 xg for 10 minutes to precipitate the Candida cells, which were then resuspended in 10 ml of phosphate-buffered saline (PBS) followed by centrifugation, to wash the cells. After this procedure was repeated once again, the cells were suspended in 10 ml of PBS and frozen for storage. Other Candida strains were treated by the same procedure as described above, to prepare antigenic materials.

### (2) Enzyme-linked immunosorbent assay (ELISA)

The above-mentioned stock cell suspension was thawed, to 2 to 3 ml of the cell suspension was added 500 ml of pure water, and the diluted cell suspension was added to an ELISA plate (Falcon (registered trade mark) 3912 Microtest III flexible assay plate, 96 wells) at an amount of 0.03 ml/well. After heating the plate at 65°C for 5 hours to evaporate liquid, 0.05 ml of 0.1% glutalaldehyde was put into each well to carry out a reaction for 10 minutes, followed by removal of the supernatant. Next, 0.15 ml of PBS supplemented with 0.1% NaN₃ and 1% bovine serum albumin (BSA) was put into each well, and after allowing to stand at 37°C for one hour, the plate was stored at 4°C, to be used as an assay plate for an anti-Candida antibody assay. Then, 60 »l of a hybridoma culture supernatant was added to this plate, which was allowed to stand at room temperature for one hour. After three times washing with PBS containing 0.05% Tween (Registered Trade Mark), 60 »l of goat anti-human IgG or IgM antibody-alkaline phosphatase (diluted 2000-fold) was added to this plate, and a reaction was carried out at room temperature for one hour. After an additional three-times washing, 100 »l of a solution prepared by dissolving P-nitrophenylphosphate in 1 M diethanolamine + 1 mM MgC1₂ (pH 9.8) at a ratio of 0.6 mg/ml was added to the plate, and after 30 to 60 minutes, the absorbance at 405 nm was measured.

### (3) Human tonsil lymphocyte

Human tonsils removed by surgery were disrupted with tweezers and scissors in ice-cold RPMI 1640 to obtain cells. The cells were twice washed by dispersing the cells in ice-cold RPMI 1640 and precipitating them by centrifugation, and finally dispersed in RPMI 1640 containing 20% FCS, 10% dimethysulfoxide and 80 »g/ml gentamycin to a concentration of about 5 x 10⁷ cells/ml. One ml each of the dispersion was put into vials and stored in liquid nitrogen. Upon use the frozen cell dispersion was thawed, and put on Ficoll-Paque (Registered Trade Mark Pharmacia), followed by centrifugation at 2000 rpm for 30 minutes to gather monocytes in an upper layer of the Ficoll Paque, and the monocytes were used as lymphocytes.

### (4) Comparison in vitro of anti-Candida antibody productivity

Lymphocytes prepared from tonsils removed from 12 patients were dispersed in culture medium A (RPMI 1640, 10% FCS, 20 mM HEPES, 2 mM glutamine, 1 mM pyruvate, 0.02 mg/ml serine and 80 »g/ml gentamycin) to a concentration of 2 x 10⁶ cells/ml, and after the addition of 20 »g/ml PWM, culturing was carried out for 6 days. The culture supernatant was obtained and assayed for anti-Candida IgG and anti-Candida IgM according to ELISA described in paragraph (2). Table 1 shows an amount of anti-Candida IgG or IgM antibody produced in vitro by each tonsil lymphocyte. The numerical values are the absorbance at 405 nm. From this result, it was found that the tonsils producing IgG antibody in a large amount were B, F and K.

**Table 1**

| Tonsil | A | B | C | D | E | F | G |
|---|---|---|---|---|---|---|---|
| IgG antibody | 0.23 | 0.61 | 0.25 | 0.32 | 0.21 | 0.71 | 0.18 |
| IgM antibody | 0.71 | 0.34 | 0.34 | 0.25 | 0.31 | 0.42 | 0.24 |

| Tonsil | H | I | J | K | L | | |
|---|---|---|---|---|---|---|---|
| IgG antibody | 0.18 | 0.14 | 0.16 | 0.73 | 0.29 | | |
| IgM antibody | 0.48 | 0.34 | 0.39 | 0.22 | 0.35 | | |

### (5) Cell fusion

Lymphocytes obtained from the tonsils B and K were dispersed in a culture medium A at a density of 2 x 10⁶ cells/ml, 10 »g/ml PWM was added to the dispersion, and culturing was carried out in 5% CO₂ at 37°C. The lymphocytes and mouse myeloma cells were subjected to cell fusion as follows. Prior to cell fusion, mouse myeloma P3 x 63 Ag8U1 cells were cultured in a culture medium (the culture medium A but excluding HEPES). 1 x 10⁷myeloma cells and 5 x 10⁶ PWM-stimulated lymphocytes were mixed, washed with a serum-free RPMI 1640, and finally centrifuged at 1500 rpm for 5 minutes to discard the supernatant. The remaining cell pellet was thoroughly disrupted by patting the test tube containing the pellet. To the test tube was added 1.0 ml polyethylene glycol solution (5.75 ml of RPMI 1640, 3.5 ml of polyethyleneglycol 1000 and 0.75 ml of dimethylsulfoxide) (abbreviated as PEG solution), and the cells were gently suspended. After one minute, 1.0 ml PBMI 1640 was added to the cell suspension, followed by additions of 2.0 ml RPMI after an additional one minute, 4.0 ml HAT medium (RPMI 1640, 10% fetal calf serum, 80 »g/ml gentamycin, 95 »M hypoxanthine, 0.4 »M aminopterin and 1.6 »M thymidine) after an additional two minutes, and 8.0 ml HAT medium after an additional two minutes, in this order. Finally, a HAT medium was added to provide a 125 ml cell suspension. The suspension was distributed to 5 culture plates (96 wells), and cultured at 37°C in 5% CO₂-containing air. At one-week intervals, a half of the culture medium was replaced with fresh HT medium (a medium the same as the HAT medium obtained by excluding A) to obtain hybridomas.

The hybridomas were screened for anti-Candida IgG productivity by ELISA, and a part of the result of the ELISA is set forth in Table 2. It is seen that hybridomas (clones) producing an anti-Candida IgG antibody were very efficiently developed.

**Table 2**

| Cell fusion plate^{(*)} | Value of ELISA (relative value of absorbance at A405 nm) | Number of clones |
|---|---|---|
| Plate No. 1 (tonsil B) | > 10 | 1 |
| | 9 to 5 | 0 |
| | 4 to 2 | 6 |
| Plate No. 2 (tonsil B) | > 10 | 0 |
| | 9 to 5 | 1 |
| | 4 to 2 | 3 |
| Plate No. 1 (tonsil K) | > 10 | 1 |
| | 9 to 5 | 0 |
| | 4 to 2 | 4 |
| Plate No. 2 (tonsil K) | > 10 | 0 |
| | 9 to 5 | 0 |
| | 4 to 2 | 5 |

| | | |
|---|---|---|
| (*) Each plate had 96 wells. | | |

Cloning was carried out by a limiting dilution technique. Namely, from anti-Candida antibody positive wells, cells were obtained, counted, and distributed to wells of a plate at a ratio of one cell/well or 10 cells/well, using a culture medium B (RPMI 1640, 10% FCS, 2 mM glutamine, 1 mM pyruvate, 0.02 mg/ml serine and 80 »g/ml gentamycin). After two weeks, the cells were sufficiently grown, and thus the supernatants were assayed to determine the presence of an anti-Candida monoclonal antibody, by ELISA, to select hybridomas capable of producing an anti-Candida monoclonal antibody.

In this manner, hybridomas PLT2-16, PML2F7 and AAE3H4 were established, and these hybridomas now stably continue to produce human monoclonal antibody.

### (7) Specificity of human anti-Candida monoclonal antibodies

It was found, by ELISA, that the human monoclonal antibodies PLT2-26 and PML2F7 are IgM antibody, and AAE3H4 is an IgG antibody, and thus their reactivity to various kinds of Candida strains were studied by ELISA. The results are shown in Table 3.

**Table 3**

| Human monoclonal antibody | Strains of Candida(*1)(*2) | | | | | | | | | |
|---|---|---|---|---|---|---|---|---|---|---|
| | 1 | 2 | 3 | 4 | 5 | 6 | 7 | 8 | 9 | 10 |
| PLT2-16 | 7 | 8 | 2 | 7 | 9 | 2 | 0 | 8 | 0 | 0 |
| PML2F7 | 5 | 6 | 3 | 9 | >10 | 0 | 0 | 0 | 0 | 0 |
| AAE3H4 | 1 | 4 | 0 | 1 | 9 | 0 | 0 | 0 | 0 | 0 |

| | | | | | | | | | | |
|---|---|---|---|---|---|---|---|---|---|---|
| (*1) Values represent result of ELISA. | | | | | | | | | | |
| (*2) Strains of Candida are as follows. | | | | | | | | | | |
| 1. C. albicans A (J 1012) 2. C. albicans B (J 1445) 3. C. tropicalis (J 1017) 4. C. guilliermondii (J 1023) 5. C. parapsilosis (J 1015) 6. C. krusei (J 1005) 7. C. kefyr (J 1004) 8. C. glabrata (J 4002) 9. Aspergillus fumigatus 10. Non addition of cells | | | | | | | | | | |

### (8) Candida antigen recognized by human MCA

Mannan from C. albicans was purified as follows. Cells were autoclaved for water extraction, and to the extract was added 75% ethanol to obtain a precipitate. The precipitate was redissolved in water, and to the solution was added Fehling's solution to obtain a precipitate, which was then dissolved in a diluted hydrochloric acid solution, and reprecipitated by 75% ethanol. The precipitate was dissolved in 3.7 N acetic acid, and to the solution was added active carbon to adsorb and eliminate impurities other than mannan. Accordingly, purified mannan was obtained from C. albicans.

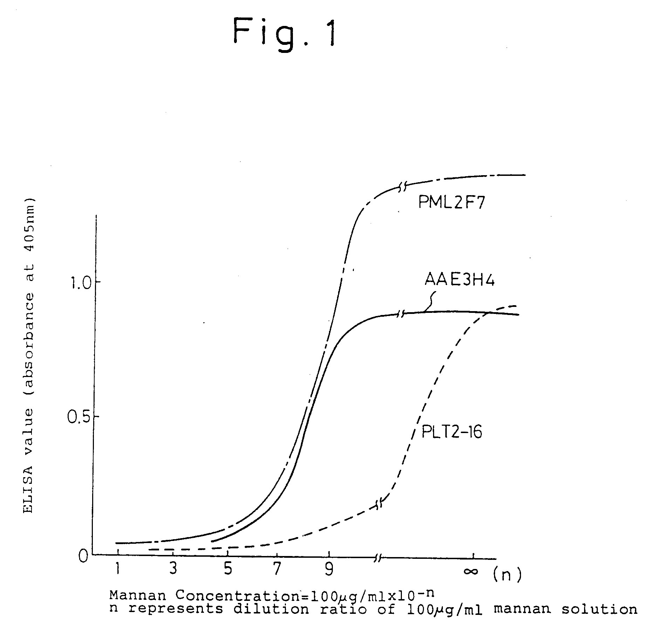

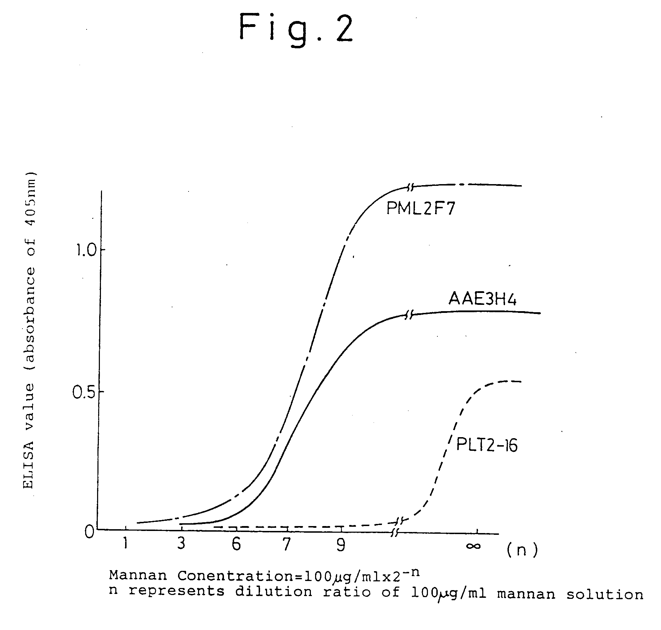

This mannan was used to test the inhibition of an antigen-antibody reaction. First, a 0.1 mg/ml mannan aqueous solution was stepwise diluted two-fold, and to the diluted solution (50 »l) was added an amount of human monoclonal antibody (59 »l), and the whole reacted at a room temperature for one hour. Then, 70 »l of the mixture was put into a well of an ELISA plate, as described in paragraph (2), to measure a reaction of a human monoclonal antibody to C. albicans A and B by ELISA. The results are shown in Figs. 1 and 2. The results show that, for all three monoclonal antibodies, i.e., AAE3H4, PLT2-16 and PML2F7, the antigen-antibody reaction is inhibited by a certain concentration of mannan or more.

### (9) Activity of human anti-Candida monoclonal antibody to agglutinate Candida cells

Living cells of Candida and formalin-treated cells prepared as described in paragraph (1) were separately suspended in PBS, and 10 »l of the suspension was thoroughly mixed with 10 »l of human monoclonal antibody (about 10 »g/ml) on a slide glass, and the agglutination was observed both microscopically and by the naked eye. The results are shown in Table 4.

**Table 4**

| Human MCA | C. albicans a | C. albicans b |
|---|---|---|
| PLT2-16 | + | + |
| PML2F7 | + | + |
| AAE3H4 | - | + |
| + : Agglutinated - : Not agglutinated | | |

From this result, it was found that these three human MCA have an ability to agglutinate Candida cells.

### (10) Purification of human monoclonal antibody

Hybridoma AAE3H4 cells were dispersed in the medium B and cultured while maintaining a cell concentration of between 1 x 10⁵ cells/ml and 10 x 10⁵ cells/ml. Then 480 ml of a resulting culture supernatant was applied to a protein A-Sepharose (Registered Trade Mark) column (size 1.2cm x 2.1cm), and after washing the column with PBS and water, a human monoclonal antibody was eluted with a diluted hydrochloric acid solution (pH 2.5). The elute was immediately neutralized by adding a one tenth volume of 10-fold concentrated PBS. Accordingly, about 4.6 mg of protein was obtained. It was confirmed by electrophoresis that this protein is a purified antibody (molecular weight about 160,000); and by ELISA that it is an antibody to C. albicans.

The above-mentioned hybridoma AAE3H4 was designated as AAE3H4(CA) and deposited with the Fermentation Research Institute Agency of Industry and Technology, 1-3 Higashi 1-chome Tsukuba-shi Ibaraki-ken Japan, as an international deposition under the Budapest Treaty, as FERM BP-2074 on September 27, 1988.

Reference to microorganism deposited under Rule 13-2 Depository Authority: Ministry of Trade and Industry, Fermentation Research Institute Agency of Industry and Technology
- Address:: 1-3 Higashi 1-chome Tsukuba-shi Ibaraki-ken Japan

## Claims

1. A hybridoma capable of producing a human monoclonal antibody against Candida, characterised in that the hybridoma is obtainable by the process comprising the steps of obtaining human B-lymphocytes, screening the lymphocytes for anti-Candida activity, selecting the lymphocytes with high anti-Candida activity, fusing said lymphocytes with myeloma cells to form hybridomas, screening the hybridomas for high antibody production and cloning said hybridomas to obtain hybridomas capable of producing anti-Candida monoclonal antibody, and in that the human monoclonal antibody produced therefrom has an ability to agglutinate Candida cells and is reactive with the Candida cell wall polysaccharide, mannan.

2. A human monoclonal antibody to Candida produced by a hybridoma according to Claim 1, characterised in that the human B-lymphocytes are selected from human tonsils, spleens, adenoids or lymph nodes.

3. A human monoclonal antibody to Candida according to Claim 2, characterised in that the B-lymphocytes are from human tonsils.

4. The human-mouse hybridoma FERM BP-2074 producing human monoclonal antibodies to Candida.

5. A human monoclonal antibody to Candida characterised in that it is produced by the deposited cell line FERM BP-2074.

## Patentansprüche

1. Hybridom, welches fähig ist, menschliche, monoklonale Antikörper gegen Candida zu bilden, dadurch gekennzeichnet, daß das Hybridom erhältlich ist durch das Verfahren, welches die Schritte umfaßt:
Gewinnen von menschlichen B-Lymphozyten,
Prüfen der Lymphozyten auf Anti-Candida-Aktivität, Auswählen der Lymphozyten mit hoher Anti-Candida-Aktivität, Verschmelzen dieser Lymphozyten mit Myelom-Zellen zur Bildung von Hybridomen,
Prüfen der Hybridome auf hohe Antikörperproduktion und Klonen der Hybridome, um Hybridome zu erhalten, welche in der Lage sind, anti-Candida monoklonale Antikörper zu bilden,
und daß der daraus hergestellte menschliche, monoklonale Antikörper eine Fähigkeit besitzt, Candida-Zellen zusammenzukleben und mit dem Candida-Zellwand-Polysaccharid Mannan zu reagieren.

2. Menschlicher monoklonaler Antikörper gegen Candida, hergestellt von einem Hybridom gemaß Anspruch 1, dadurch gekennzeichnet, daß die menschlichen B-Lymphozyten ausgewählt sind von menschlichen Mandeln, Milz, Adenoiden oder Lymphknoten.

3. Menschlicher, monoklonaler Antikörper gegen Candida gemäß Anspruch 2, dadurch gekennzeichnet, daß die B-Lymphozyten aus menschlichen Mandeln stammen.

4. Menschen/Maus-Hybridom FERM BP-2074, welches menschliche monoklonale Antikörper gegen Candida bildet.

5. Menschlicher monoklonaler Antikörper gegen Candida, dadurch gekennzeichnet, daß er von der hinterlegten Zellinie FERM BP-2074 gebildet ist.

## Revendications

1. Hybridome capable de produire un anticorps monoclonal humain dirigé contre Candida, caractérisé en ce que l'hybridome peut être obtenu par le procédé comprenant les étapes consistant à obtenir des lymphocytes B humains, à cribler les lymphocytes en fonction de leur activité anti-Candida, à sélectionner les lymphocytes présentant une activité anti-Candida élevée, à fusionner lesdits lymphocytes avec des cellules de myélome pour former des hybridomes, à cribler les hybridomes en fonction de leur production élevée d'anticorps et à cloner lesdits hybridomes pour obtenir des hybridomes capables de produire un anticorps monoclonal anti-Candida, et en ce que l'anticorps monoclonal humain ainsi produit a la capacité d'agglutiner les cellules de Candida et réagit avec le polysaccharide de la paroi cellulaire de Candida, le mannane.

2. Anticorps monoclonal humain anti-Candida produit par un hybridome selon la revendication 1, caractérisé en ce que les lymphocytes B humains sont sélectionnés parmi des amygdales, des rates, des végétations adénoïdes ou des ganglions lymphatiques humains.

3. Anticorps monoclonal humain anti-Candida selon la revendication 2, caractérisé en ce que les lymphocytes B proviennent d'amygdales humaines.

4. Hybridome humain-souris FERM BP-2074 produisant des anticorps monoclonaux humains anti-Candida.

5. Anticorps monoclonal humain anti-Candida, caractérisé en ce qu'il est produit par la lignée cellulaire déposée FERM BP-2074.
